Europäisches Patentamt

European Patent Office

Office européen des brevets

(11) Veröffentlichungsnummer: **0 210 185 B1**

(12) **EUROPÄISCHE PATENTSCHRIFT**

(45) Veröffentlichungstag der Patentschrift:
**16.08.89**

(21) Anmeldenummer: **86900120.6**

(22) Anmeldetag: **11.12.85**

(86) Internationale Anmeldenummer:
**PCT/EP 85/00695**

(87) Internationale Veröffentlichungsnummer:
**WO 86/04484 (14.08.86 Gazette 86/18)**

(51) Int. Cl.⁴: **A 01 M 13/00,** A 01 M 1/20,
A 61 L 9/03

(54) **VORRICHTUNG ZUM VERDAMPFEN VON IN ZELLSTOFF ODER ANDEREN TRäGERMATERIALIEN EINGELAGERTEN WIRKSTOFFEN.**

(30) Priorität: **30.01.85 DE 8502409 U**

(43) Veröffentlichungstag der Anmeldung:
**04.02.87 Patentblatt 87/6**

(45) Bekanntmachung des Hinweises auf die Patenterteilung:
**16.08.89 Patentblatt 89/33**

(84) Benannte Vertragsstaaten:
**AT BE CH DE FR GB IT LI NL SE**

(56) Entgegenhaltungen:
**DE-B- 2 730 855**
**FR-A- 2 183 121**
**GB-A- 937 866**
**GB-A- 2 080 111**

(73) Patentinhaber: **Globol-Werk GmbH,**
**Anna-von-Philipp-Strasse 33, D-8858 Neuburg/Donau (DE)**

(72) Erfinder: **VON PHILIPP, Fritz, Auf der Klause 32 1/3,**
**D-8858 Neuburg/Donau (DE)**
Erfinder: **HAUTMANN, Horst, Flachslandenstrasse 11,**
**D-8858 Neuburg/Donau (DE)**

(74) Vertreter: **Reinhard, Skuhra, Weise, Leopoldstrasse 51,**
**D-8000 München 40 (DE)**

ACTORUM AG

## Beschreibung

Die Erfindung betrifft eine Vorrichtung gemäß dem Oberbegriff des Patentanspruchs 1.

Eine Vorrichtung nach dem Oberbegriff des Patentanspruchs 1 ist aus der DE-B-2 730 855 bekannt. Bei dieser Vorrichtung hat der Wirkstoffträger plattenförmige Gestalt und wird zur Verdampfung der Wirkstoffe über seine gesamte Fläche an die auf Betriebstemperatur erwärmte und hinter einem Gehäusefenster angeordnete elektrische Heizung angelegt. Hierdurch wird eine intensive Verdampfung von Wirkstoffen erreicht. Je intensiver aber die Verdampfung ist, um so rascher erschöpft sich der in den Wirkstoffträger eingelagerte Vorrat an Wirkstoffen, so daß der Wirkstoffträger nach relativ kurzer Zeit ausgetauscht werden muß, wenn über einen längeren Zeitraum eine konstante Verdampfung an Wirkstoffen erwünscht ist.

Der Erfindung liegt die Aufgabe zugrunde, eine Vorrichtung nach dem Oberbegriff des Patentanspruchs 1 derart zu verbessern, daß mit einfachen Mitteln und Maßnahmen über einen weit längeren Zeitraum als mit der bekannten Vorrichtung eine den Umständen entsprechend richtige Dosierung bei der Verdampfung von Wirkstoffen erreichbar ist, ohne das Erfordernis des Austausches des Wirkstoffträgers.

Diese Aufgabe wird erfindungsgemäß durch die im kennzeichnenden Teil des Patentanspruchs 1 angegebenen Merkmale gelöst.

Weitere Ausgestaltungen ergeben sich aus den Unteransprüchen.

Bei der erfindungsgemäßen Vorrichtung steht der elektrischen Heizung eine zum Volumen des Wirkstoffträgers relativ kleine Fläche gegenüber, die zur Verdampfung von Wirkstoffen von der Heizung beeinflußt wird. Hieraus resultiert einerseits eine wohldosierte Abgabe an Wirkstoffen, andererseits aber auch die Möglichkeit einer Erhöhung der Kapazität des Wirkstoffträgers, ohne die von der Heizung beeinflußbare Fläche des Wirkstoffträgers zu vergrößern. Die verdampften Wirkstoffe werden durch die von der Heizung erwärmte und durch den von der Hülse und dem Gehäusemantel begrenzten Abgaskanal nach oben strömende Raumluft angesaugt und mit relativ hoher Geschwindigkeit mitgenommen, woraus eine rasche und gleichmäßige Verteilung der verdampften Wirkstoffe im Raum resultiert.

Die Anordnung der Hülse mit dem Wirkstoffträger oberhalb der Heizung ermöglicht die Verwendung von weniger warmfesten und preiswerteren Wirkstoffen für Hülse und Wirkstoffträger.

Eine vorteilhafte Weiterbildung der Vorrichtung besteht darin, daß der Abstand zwischen der den Wirkstoffträger enthaltenden Hülse gegenüber der Heizung einstellbar ausgebildet ist. Hierdurch läßt sich durch Veränderung des Abstandes zwischen Hülse mit Wirkstoffträger und Heizung die Intensität der Verdampfung so ändern, daß auch bei längerer Betriebsdauer eine gleichbleibende Verdampfung an Wirkstoff erreichbar ist.

Bei einer vorteilhaften Ausbildung der Vorrichtung ist zur Erhöhung der Konvektion die Hülse und der Wirkstoffträger mittig über der Heizung angeordnet und die Abstrahlfläche der Heizung ist größer als die der Heizung zugewandte Stirnseite des Wirkstoffträgers.

Im folgenden werden bevorzugte Ausführungsformen der Vorrichtung anhand der Zeichnung beschrieben. Es zeigen:

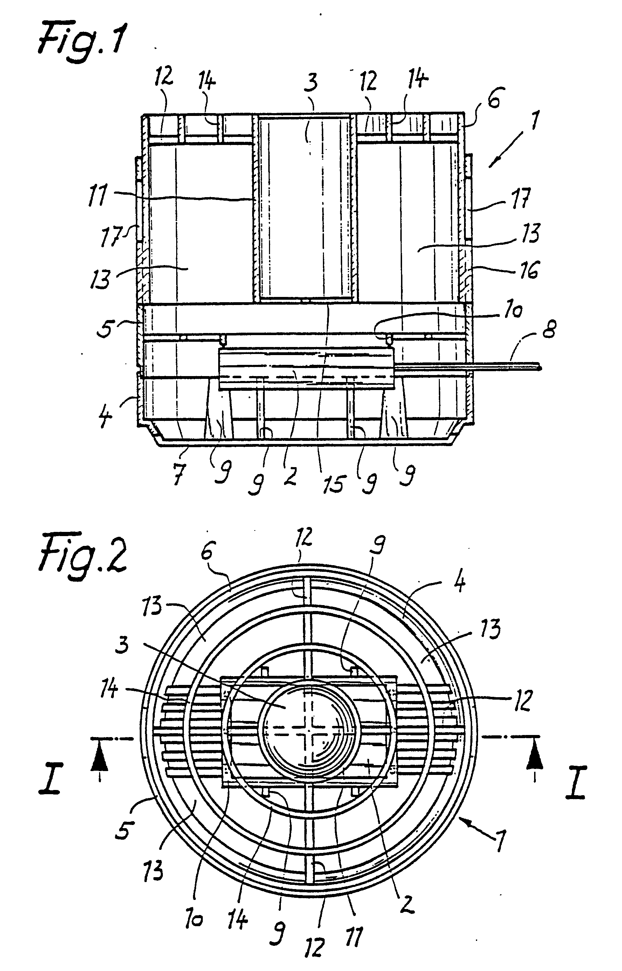

Fig. 1 eine in Schnittansicht entlang der Schnittlinie I-I in Fig. 2 gezeigte Vorrichtung,

Fig. 2 eine Aufsicht auf die Vorrichtung nach Fig. 1,

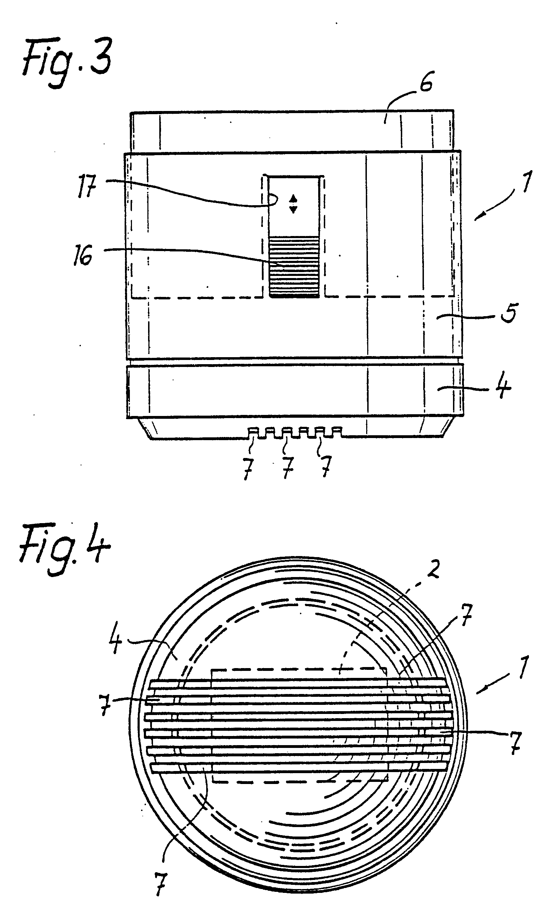

Fig. 3 eine Seitenansicht der Vorrichtung, und

Fig. 4 eine Ansicht der Vorrichtung von unten.

Die in den Zeichnungen gezeigte Vorrichtung weist ein aus formfestem Kunststoff hergestelltes Gehäuse 1 auf, innerhalb welchem eine elektrische Heizung 2 und ein austauschbar angeordneter, zylinderförmiger Wirkstoffträger 3 aus Zellstoff vorgesehen sind. Das Gehäuse ist aus einem schalenförmigen Basisteil 4, einem darüber angeordneten Mittelteil 5 und einem Oberteil 6 zusammengefügt. Diese Teile 4 bis 6 sind kreisringförmig profiliert, zueinander gleichachsig und lotrecht übereinander angeordnet.

Der Mantel des Mittelteiles 5 hat den gleichen Durchmesser wie der Mantel des Basisteiles 4 und das Oberteil 6 ist in das Mittelteil 5 mit Haftreibung axial verstellbar eingesteckt.

Im Boden des Basisteiles 4 sind schlitzförmige Belüftungsöffnungen angeordnet, die bis in den Mantel des Basisteiles 4 hineinreichen.

Zwischen dem Basisteil 4 und dem Mittelteil 5 ist die quaderförmige Heizung so eingespannt, daß die Breitseiten der elektrischen Heizung 2 waagerecht gerichtet sind.

Die elektrische Zuleitung 8 der elektrischen Heizung ist zur Bildung einer Zugentlastung zwischen den einander zugewandten Stirnseiten der Gehäuseteile 4 und 5 eingeklemmt. Anstelle der dargestellten Zuleitung 8 können am Gehäuse 1 auch zwei nicht dargestellte elektrische Steckkontakte quer abstrebend gehaltert werden, die mit der elektrischen Heizung 2 elektrisch leitend zu verbinden sind.

Zum Einspannen der Heizung 2 sind am Basisteil 4 von dessen Boden vertikal nach oben abstrebende Wände 9 und im Mittelteil 5 ein Rahmen 10 angeformt.

Letzterer ist durch Streben mit dem Mantel des Mittelteiles 5 verbunden.

In dem über dem Rahmen 10 befindlichen zylindrischen Teil des Gehäuse-Mittelteiles 5 ist der zylindrische Gehäuse-Oberteil 6 eingesetzt, in dem zu letzterem koaxial ausgerichtet eine Hülse 11 angeformt ist, die über radial gerichtete, angeformte Wände 12 mit dem Mantel des Gehäuseteiles 6 stoffschlüssig verbunden ist.

Der vom Mantel des Oberteiles 6 und der Hülse 11 umgrenzte Abgaskanal 13 ist im Bereich der oberen Mündung mit einem Gitter 14 so verschlossen, daß er mit den Fingern einer menschlichen Hand nicht durchgriffen werden kann.

An der unteren Mündung der Hülse 11 sind noch die Einstecktiefe des Wirkstoffträgers 3 begrenzende Anschläge 15 in Form zweier sich kreuzender Querstäbe angeformt.

Zur bequemen Verstellung des Oberteiles 6 relativ zum Mittelteil 5 sind am Oberteil 6 zwei diametral gegenüber angeordnete, nach innen elastisch durchfedernde Handhaben 16 angeformt, die in im Mittelteil 5 angeordnete, achsparallel und vertikal gerichtete, den Handhaben 16 angepaßte Schlitze höhenverstellbar durchgreifen.

Der maximale Hub des Oberteiles 6 beträgt etwa 1,5 cm und die Hülse 11 samt Wirkstoffträger 3 ist von der elektrischen Heizung mit einem lotrechten Abstand von ca. 0,5 cm angeordnet.

Der Wirkstoffträger ist in die umfangsseitig geschlossene Hülse 11 mit Haftreibung eingesteckt. Hat die Heizung ihre Betriebstemperatur von etwa 130°C erreicht, wird dadurch zwangsläufig die Unterseite des Wirkstoffträgers 3 so erwärmt, daß die im Wirkstoffträger 3 eingekapselten Wirkstoffe verdampfen und von der erwärmten, durch den Abgaskanal 13 nach oben durchströmenden Raumluft mitgenommen werden.

Um so näher der Wirkstoffträger 3 der elektrischen Heizung 2 angeordnet ist, um so größer ist auch die Erwärmung der Wirkstoffe und um so mehr verdampfen diese.

Mit dem wie dargestellt bemessenen Wirkstoffträger 3, der einen Durchmesser von ca. 2 cm und eine axiale Länge von etwa 3 cm oder mehr aufweist, und in den ca. 4,5 g Wirkstoff eingelagert ist, kann eine konstante Verdampfung von Wirkstoff über ca. 8 Tage erreicht werden, wozu anfangs der Wirkstoffträger 3 mit großem Abstand von der elektrischen Heizung anzuordnen ist und mit zunehmender Dauer der Verdampfung immer mehr der Heizung genähert werden muß.

Durch eine konische Verengung des Abzugskanals 13 nach oben, kann die Ausströmungsgeschwindigkeit der erwärmten Luft und somit auch der verdampften Wirkstoffe vergrößert werden.

Zusätzlich zur höhenverstellbaren Anordnung des Wirkstoffträgers zur Veränderung des Verdampfungsgrades oder anstelle dieser kann auch eine in ihrer Betriebstemperatur veränderbare Heizung vorgesehen werden, die aber einerseits wesentlich aufwendiger als eine in ihrer Betriebstemperatur konstante Heizung ist und andererseits bei überhöhter Betriebstemperatur über 130°C hinaus unter anderem die Funktion der Vorrichtung gefährden könnte.

Unter Umständen könnte es auch vorteilhaft sein, mehrere Wirkstoffträger erfindungsgemäß über einer gemeinsamen Heizung zu haltern.

Alle neuen, in der Beschreibung und/oder Zeichnung offenbarten Einzel- und Kombinationsmerkmale werden als erfindungswesentlich angesehen.

Bei einer bevorzugten Ausführungsform der Vorrichtung ist der zylindrische Behälter 3 zur Aufnahme des vorzugsweise porösen Wirkstoffträgers vorgesehen und in Richtung auf die Heizplatte 2 durch einen Boden abgeschlossen. Besteht der Behälter 3 aus Metall oder einem anderen gut wärmeleitenden Material, wirkt die von der Heizplatte 2 abgestrahlte Wärme auf sämtliche Wand- und Bodenabschnitte des Behälters 3 und führt zu einer intensiven aber gleichmäßigen Verdampfung der Wirkstoffe.

Nach einer weiteren Ausführungsform ist vorgesehen, daß die den zylindrischen Behälter 3 aufnehmende Hülse 11 bereichsweise, d.h. umfangsmäßig ausgebildete Aussparungen aufweist, wodurch die von der Heizplatte 2 wegströmende Warmluft über die Aussparungen auf den beispielsweise gut wärmeleitfähigen zylindrischen Behälter 3 wirkt und den Behälter 3 über seine gesamte Länge bzw. Höhe weitgehend gleichmäßig beaufschlagt. Dadurch wird die Verdampfung der Wirkstoffe auch bereits bei niedrigeren Temperaturen wirksam erreicht.

## Patentansprüche

1. Vorrichtung zum Verdampfen von in Zellstoffen oder einem anderen, mindestens annähernd formfesten Trägermaterial eingelagerten Wirkstoffen, bestehend aus einem Gehäuse, in welchem eine elektrische Heizung zur Verdampfung der Wirkstoffe angeordnet ist, mit einer den Wirkstoffträger über der Heizung austauschbar positionierenden Halterung und mit im unteren Bereich des Gehäuses ausgebildeten Belüftungsöffnungen, dadurch gekennzeichnet, daß der Wirkstoffträger (3) zylindrische Gestalt hat, daß die Halterung (11) oberhalb der Heizung (2) und zur Heizung (2) beabstandet in Form einer oben und unten offenen Hülse (11) vorgesehen ist und den zylindrischen Wirkstoffträger (3) unter Einhaltung des Abstandes zur Heizung aufnimmt, und daß die Hülse (11) innerhalb des nur oben Abgasöffnungen aufweisenden Gehäuses (1) mit allseitigem Abstand vom Gehäusemantel angeordnet ist.

2. Vorrichtung nach Anspruch 1, dadurch gekennzeichnet, daß der Abstand zwischen Hülse (11) mit Wirkstoffträger (3) gegenüber der Heizung (2) einstellbar ausgebildet ist.

3. Vorrichtung nach Anspruch 1 oder 2, dadurch gekennzeichnet, daß die Hülse (11) mittig über der Heizung (2) angeordnet ist, und daß die Heizung (2) eine Abstrahlfläche aufweist, welche größer ist als die der Heizung (2) zugewandte Stirnseite des Wirkstoffträgers (3).

4. Vorrichtung nach Anspruch 1 oder 2, dadurch gekennzeichnet, daß die Hülse (11) mittig zum Gehäusemantel vorgesehen ist.

5. Vorrichtung nach Anspruch 1, dadurch gekennzeichnet, daß die Hülse (11) und der Gehäusemantel eine im wesentlichen gleiche Querschnittsform unterschiedlicher Dimensionen aufweisen.

6. Vorrichtung nach Anspruch 5, dadurch gekennzeichnet, daß die Hülse (11) und der Gehäusemantel zueinander gleichachsig vorgesehen sind.

7. Vorrichtung nach einem der Ansprüche 1 bis 6, dadurch gekennzeichnet, daß die Hülse (11) und der Wirkstoffträger (3) jeweils über ihre gesamte Länge gleiche Querschnitte aufweisen.

8. Vorrichtung nach einem der Ansprüche 1 bis 7, dadurch gekennzeichnet, daß sich der lichte Querschnitt der Hülse (11) zum lichten Querschnitt des Gehäusemantels im Verhältnis von etwa 1 : 5 verhält.

9. Vorrichtung nach einem der Ansprüche 1 bis 8, dadurch gekennzeichnet, daß der Gehäusemantel im lichten Querschnitt sich nach oben verjüngend ausgebildet ist.

10. Vorrichtung nach einem der Ansprüche 1 bis 9, dadurch gekennzeichnet, daß die lichte Weite der Hülse (11) etwa 2/5 der Länge der Hülse (11) entspricht.

11. Vorrichtung nach einem der Ansprüche 1 bis 10, dadurch gekennzeichnet, daß die Belüftungsöffnungen (7) im Gehäuse (1) mit einem von der Hülse (11) und dem Gehäusemantel festgelegten Abgaskanal (13) in Verbindung stehen.

12. Vorrichtung nach Anspruch 11, dadurch gekennzeichnet, daß der von der Hülse (11) und dem Gehäusemantel festgelegte Abgaskanal (13) vertikal verläuft und vergittert ist.

13. Vorrichtung nach einem der Ansprüche 1 bis 12, dadurch gekennzeichnet, daß der Hülse (11) ein diese oben verschließbarer Deckel zugeordnet ist.

14. Vorrichtung nach einem der Ansprüche 1 bis 13, dadurch gekennzeichnet, daß der Wirkstoffträger (3) in die Hülse (11) reibschlüssig gesichert eingesteckt ist.

15. Vorrichtung nach einem der Ansprüche 1 bis 14, dadurch gekennzeichnet, daß am unteren Ende der Hülse (11) nach innen vorstehende, zur Begrenzung der Einstecktiefe des Wirkstoffträgers (3) vorgesehene Anschläge (15) angeordnet sind.

16. Vorrichtung nach einem der Ansprüche 1 bis 15, dadurch gekennzeichnet, daß am Wirkstoffträger (3) eine oben aus der Hülse (11) herausragende Handhabe, insbesondere ein Band oder ein Faden, angeordnet ist.

17. Vorrichtung nach einem der Ansprüche 1 bis 16, dadurch gekennzeichnet, daß das Gehäuse (1) aus einem die Belüftungsöffnungen (7) aufweisenden, schalenförmigen Basisteil (4), einem darüber angeordneten Mittelteil (5) und einem, an dem Mittelteil teleskopartig lotrecht einstellbar gehaltertem Oberteil (6), zusammengesetzt ist, daß die Hülse (11) im Oberteil (6) angeordnet, insbesondere angeformt ist, und daß zwischen dem Basisteil (4) und dem Mittelteil (5) die Heizung (2) eingespannt ist.

18. Vorrichtung nach Anspruch 17, dadurch gekennzeichnet, daß das Oberteil (6) mit Haftreibung in das Mittelteil (5) eingesteckt ist, und daß am Oberteil (6) mindestens eine, insbesondere zwei, diametral einander gegenüber angeordnete und entsprechende, im wesentlichen lotrecht verlaufende Schlitze (17) im Mittelteil (5) durchgreifende Handhaben (16) angeordnet sind.

## Claims

1. Apparatus for evaporating active substances included in pulp or cellulose or another at least substantially dimensionally stable carrier material, consisting of a housing in which an electrical heating means for vaporizing the active substances is arranged, a holding means positioning the active substance carrier replaceably over the heating means and ventilation openings formed in the lower region of the housing, characterized in that the active substance carrier (3) has a cylindrical shape, that the holding means (11) is provided above the heating means (2) and spaced from said heating means (2) in the form of a sleeve (11) open at the top and bottom and receives the cylindrical active substance carrier (3) whilst retaining the distance from the heating means, and that the sleeve (11) is arranged within the housing (1) having exhaust gas openings only at the top in all round spaced relationship with the housing jacket.

2. Apparatus according to claim 1, characterized in that the spacing between the sleeve (11) with active substance carrier (3) from the heating means (2) is adjustable.

3. Apparatus according to claim 1 or 2, characterized in that the sleeve (11) is disposed centrally over the heating means (2) and that the heating means (2) comprises an irradiation surface which is larger than the end face of the active substance carrier (3) facing the heating means (2).

4. Apparatus according to claim 1 or 2, characterized in that the sleeve (11) is provided centrally with respect to the housing jacket.

5. Apparatus according to claim 1, characterized in that the sleeve (11) and the housing jacket have a substantially identical cross-sectional form of different dimensions.

6. Apparatus according to claim 5, characterized in that the sleeve (11) and the housing jacket are provided coaxially to each other.

7. Apparatus according to any one of claims 1 to 6, characterized in that the sleeve (11) and the active substance carrier (3) each have the same cross-section over their entire length.

8. Apparatus according to any one of claims 1 to 7, characterized in that the internal clear cross-section of the sleeve (11) is in a ratio of about 1 : 5 to the internal clear cross-section of the housing jacket.

9. Apparatus according to any one of claims 1 to 8, characterized in that the housing jacket in internal clear cross-section is made upwardly tapering.

10. Apparatus according to any one of claims 1 to 9, characterized in that the internal width of the sleeve (11) is about 2/5 the length of the sleeve (11).

11. Apparatus according to any one of claims 1 to 10, characterized in that the ventilation openings (7) in the housing (1) communicate with an exhaust gas passage (13) defined by the sleeve (11) and the housing jacket.

12. Apparatus according to claim 11, characterized in that the exhaust gas passage (13) defined by the sleeve (11) and housing jacket extends vertically and is provided with a grating.

13. Apparatus according to any one of claims 1 to 12, characterized in that the sleeve (11) comprises a cover closing said sleeve at the top.

14. Apparatus according to any one of claims 1 to 13, characterized in that the active substance carrier (3) is inserted into the sleeve (11) and secured by friction.

15. Apparatus according to any one of claims 1 to 14, characterized in that at the lower end of the sleeve (11) inwardly projecting stops (15) are provided for limiting the insertion depth of the active substance carrier (3).

16. Apparatus according to any one of claims 1 to 15, characterized in that on the active substance carrier (3) a grip, in particular a strap or cord, projecting upwardly from the sleeve (11) is disposed.

17. Apparatus according to any one of claims 1 to 16, characterized in that the housing (1) is made up of a dish-like base part (4) comprising the ventilation openings (7), a centre part (5) disposed thereabove and an upper part (6) which is mounted telescopically vertically adjustably on the centre part, that the sleeve (11) is arranged, in particular integrally formed, in the upper part (6) and that the heating means (2) is clamped between the base part (4) and the centre part (5).

18. Apparatus according to claim 17, characterized in that the upper part (6) is inserted with static friction into the centre part (5) and that on the upper part (6) at least one, in particular two, diametrically oppositely disposed grips (16) passing through correspondingly substantially perpendicularly extending slots (17) in the centre part (5) are disposed.

## Revendications

1. Dispositif de vaporisation de substances actives emmagasinées dans une matière alvéolaire ou un autre matériau de support de forme au moins approximativement stable, constitué d'un boîtier dans lequel est disposé un chauffage électrique pour vaporiser les substances actives et qui comporte un support, maintenant le porte-substances actives au-dessus du chauffage d'une manière lui permettant d'être remplacé, et des orifices de prise d'air ménagés dans la partie inférieure du boîtier, caractérisé en ce que le porte-substances actives (3) à une forme cylindrique,

en ce que le support (11) est prévu, au-dessus du chauffage (2) et espacé de celui-ci, sous la forme d'un manchon (11) ouvert à sa partie supérieure et à sa partie inférieure, et reçoit le porte-substances actives cylindrique (3) en maintenant son espacement par rapport au chauffage

et en ce que le manchon (11) est situé à l'intérieur du boîtier (1), qui ne présente des orifices pour effluents gazeux qu'à sa partie supérieure, en étant espacé de tous côtés de l'enveloppe cylindrique de ce boîtier.

2. Dispositif suivant la revendication 1, caractérisé en ce que la distance entre le manchon (11), avec le porte-substances actives (3), et le chauffage (2) est réglable.

3. Dispositif suivant la revendication 1 ou 2, caractérisé en ce que le manchon (11) est situé en position centrale au-dessus du chauffage (2) et en ce que le chauffage (2) comporte une surface radiante qui est plus grande que la surface frontale, tournée vers le chauffage (2), du porte-substances actives (3).

4. Dispositif suivant la revendication 1 ou 2, caractérisé en ce que le manchon (11) est prévu en position centrale par rapport à l'enveloppe cylindrique du boîtier.

5. Dispositif suivant la revendication 1, caractérisé en ce que le manchon (11) et l'enveloppe cylindrique du boîtier possèdent, en section transversale, des formes pratiquement identiques de dimensions différentes.

6. Dispositif suivant la revendication 5, caractérisé en ce que le manchon (11) et l'enveloppe cylindrique du boîtier sont coaxiaux l'un à l'autre.

7. Dispositif suivant l'une des revendications 1 à 6, caractérisé en ce que le manchon (11) et le porte-substances actives (3) possèdent chacun des sections transversales identiques sur toute leur longueur.

8. Dispositif suivant l'une des revendications 1 à 7, caractérisé en ce que la section transversale intérieure du manchon (11) est dans un rapport d'environ 1 : 5 par rapport à la section transversale intérieure de l'enveloppe cylindrique du boîtier.

9. Dispositif suivant l'une des revendications 1 à 8, caractérisé en ce que l'enveloppe cylindrique du boîtier possède une section transversale intérieure qui se rétrécit vers le haut.

10. Dispositif suivant l'une des revendications 1 à 9, caractérisé en ce que la largeur intérieure du manchon (11) correspond à environ 2/5 de sa longueur.

11. Dispositif suivant l'une des revendications 1 à 10, caractérisé en ce que les orifices de prise d'air (7) du boîtier (1) communiquent avec un conduit d'effluents gazeux (13) déterminé par le manchon (11) et l'enveloppe cylindrique du boîtier.

12. Dispositif suivant la revendication 11, caractérisé en ce que le conduit d'effluents gazeux (13) déterminé par le manchon (11) et l'enveloppe cylindrique du boîtier est orienté verticalement et est pourvu d'une grille.

13. Dispositif suivant l'une des revendications 1 à 12, caractérisé en ce qu'il est associé au manchon (11) un couvercle pouvant le fermer à sa partie supérieure.

14. Dispositif suivant l'une des revendications 1 à 13, caractérisé en ce que le porte-substances actives (3) est emboîté dans le manchon (11) en y étant immobilisé par frottement.

15. Dispositif suivant l'une des revendications 1 à 14, caractérisé en ce que, à l'extrémité inférieure du manchon (11), il est prévu des butées (15), qui font saillie vers l'intérieur et servent à limiter la profondeur d'emboîtement du porte-substances actives (3).

16. Dispositif suivant l'une des revendications 1 à 15, caractérisé en ce que, sur le porte-substances actives (3), il est prévu un élément de manipulation, notamment un ruban ou un cordon, faisant saillie vers le haut hors du manchon (11).

17. Dispositif suivant l'une des revendications 1 à 16, caractérisé en ce que le boîtier (1) est composé d'une partie de base (4), en forme de cuvette, comportant des orifices de prise d'air (7), d'une partie centrale (5) disposée au-dessus de celle-ci et d'une partie supérieure (6) maintenue de manière télescopique sur la partie centrale, en étant réglable verticalement en position, en ce que le manchon (11) est disposé dans la partie supérieure (6), notamment en étant réalisé d'une seule pièce avec celle-ci, et en ce que le chauffage (2) est immobilisé par serrage entre la partie de base (4) et la partie centrale (5).

18. Dispositif suivant la revendication 17, caractérisé en ce que la partie supérieure (6) est emboîtée avec adhérence par frottement dans la partie centrale (5) et en ce que, sur la partie supérieure (6), il est prévu au moins un et notamment deux éléments de manipulation (16) qui sont disposés de manière diamétralement opposée l'un à l'autre et traversent des fentes (17), orientées de manière essentiellement verticale, de la partie centrale (5).

*Fig.1*

*Fig.2*

*Fig.3*

*Fig.4*